# EUROPEAN PATENT SPECIFICATION

(11) **EP 3 956 471 B1**
(45) Date of publication and mention of the grant of the patent: **30.10.2024**
(21) Application number: 20719759.1
(22) Date of filing: 17.04.2020
(51) Int. Cl.: C12Q 1/6876

(54) **A METHOD TO PREDICT THE PREDISPOSITION TO AN EXERCISE PERFORMANCE TRAIT IN A HUMAN INDIVIDUAL**
VERFAHREN ZUR VORHERSAGE DER PRÄDISPOSITION FÜR EIN ÜBUNGSLEISTUNGSMERKMAL BEI EINEM MENSCHLICHEN INDIVIDUUM
PROCÉDÉ POUR PRÉDIRE LA PRÉDISPOSITION À UN TRAIT DE PERFORMANCE POUR L'EXERCICE CHEZ UN INDIVIDU HUMAIN

(30) Priority: 19.04.2019 IT 201900006196
(43) Date of publication of application: 23.02.2022
(73) Proprietor: Sbarro Health Research Organization Inc., Radnor, PA 19087 (US)
(72) Inventor: GIORDANO, Antonio, Radnor, Pennsylvania 19087 (US); LA MONTAGNA, Raffaele, Radnor, Pennsylvania 19087 (US); BUCCI, Enrico, Radnor, Pennsylvania 19087 (US); BOFFO, Silvia, Radnor, Pennsylvania 19087 (US); DE NICOLA, Alfonso, Radnor, Pennsylvania 19087 (US)
(74) Representative: Studio Torta S.p.A.
(86) International application number: PCT/IB2020/053667
(87) International publication number: WO 2020/212936

(56) References cited:
- WO-A1-2011/094815
- WO-A1-2018/036495
- NICHOLAS JONES ET AL: "A genetic-based algorithm for personalized resistance-training", BIOLOGY OF SPORT, vol. 33, no. 2, 6 March 2016 (2016-03-06), pages 117 - 126, XP055388335, ISSN: 0860-021X, DOI: 10.5604/20831862.1198210
- EDUARDO M. PIMENTA ET AL: "Effect of ACTN3 Gene on Strength and Endurance in Soccer Players :", JOURNAL OF STRENGTH AND CONDITIONING RESEARCH, vol. 27, no. 12, 1 December 2013 (2013-12-01), US, pages 3286 - 3292, XP055654676, ISSN: 1064-8011, DOI: 10.1519/JSC.0b013e3182915e66
- PICKERING CRAIG ET AL: "Hamstring injury prevention: A role for genetic information?", MEDICAL HYPOTHESES, EDEN PRESS, PENRITH, US, vol. 119, 1 August 2018 (2018-08-01), pages 58 - 62, XP085452452, ISSN: 0306-9877, DOI: 10.1016/J.MEHY.2018.07.011
- MYOSOTIS MASSIDDA ET AL: "ACTN3 R577X Polymorphism Is Associated With the Incidence and Severity of Injuries in Professional Football Players :", CLINICAL JOURNAL OF SPORT MEDICINE, vol. 29, no. 1, 1 January 2019 (2019-01-01), US, pages 57 - 61, XP055654679, ISSN: 1050-642X, DOI: 10.1097/JSM.0000000000000487
- MYOSOTIS MASSIDDA ET AL: "Influence of the MCT1 rs1049434 on Indirect Muscle Disorders/Injuries in Elite Football Players", SPORTS MEDICINE - OPEN, vol. 1, no. 1, 11 October 2015 (2015-10-11), XP055654683, ISSN: 2199-1170, DOI: 10.1186/s40798-015-0033-9
- MASOUDA RAHIM ET AL: "Human Genetic Variation, Sport and Exercise Medicine, and Achilles Tendinopathy: Role for Angiogenesis-Associated Genes", OMICS A JOURNAL OF INTEGRATIVE BIOLOGY, vol. 20, no. 9, 1 September 2016 (2016-09-01), NEW YORK, NY, US, pages 520 - 527, XP055654776, ISSN: 1536-2310, DOI: 10.1089/omi.2016.0116
- J L CHICHARRO: "Mutations in the hereditary haemochromatosis gene HFE in professional endurance athletes", BRITISH JOURNAL OF SPORTS MEDICINE, vol. 38, no. 4, 1 August 2004 (2004-08-01), GB, pages 418 - 421, XP055654684, ISSN: 0306-3674, DOI: 10.1136/bjsm.2002.003921
- AFFYMETRIX: "Data Sheet Affymetrix(R) Genome-Wide Human SNP Array 6.0", INTERNET CITATION, 1 January 2007 (2007-01-01), pages 1 - 4, XP002525407, Retrieved from the Internet <URL:http://www.affymetrix.com/support/technical/datasheets/genomewide_snp6_datasheet.pdf> [retrieved on 20090410]
- RAFFAELE MONTAGNA ET AL: "Genomic analysis reveals association of specific SNPs with athletic performance and susceptibility to injuries in professional soccer players", JOURNAL OF CELLULAR PHYSIOLOGY, vol. 235, no. 3, 7 August 2019 (2019-08-07), US, pages 2139 - 2148, XP055654706, ISSN: 0021-9541, DOI: 10.1002/jcp.29118

## Description

### Technical Field of the Invention

The present invention relates to a method to predict the predisposition to an exercise performance trait in a human individual comprising assaying for the presence of polymorphisms in each of the following genes: ACTN3, COL5A1, MCT1, VEGF, HFE.

### Prior Art

Training for elite athletes is a complex task that needs to be adapted not only to specific sports but also to the individual predisposition of each single athlete. Among others, soccer is considered a total and very demanding sport that requires the concomitant training of different body systems, such as muscular, cardio-circulatory, respiratory and nervous system often supported by specific nutrients supplementation. This multi-parametric training fulfils different physical and nutritional requirements that, in turn, might improve muscle size and strength, endurance, resistance to fatigue, and coordination.

Although the physical and metabolic features of each athlete are considered to be the main parameters to design specific training programs, it is currently accepted the impact of genetic traits on the success of a given training. Advances in science and technology could in principle change the way in which soccer players are subjected to training programs designed in a personalized perspective. The inclusion of genetics in the design of training programs (PEP, Performance Enhancing Polymorphisms) was triggered by the identification of a large number of genetic variants, linked to the athletic performances (Ostrander et al., 2009) .

The correlation between specific gene polymorphisms and the response to physical and nutritional cues can then be explored to evaluate athlete's profiles. This would allow the development of tailored programs that maximize the athletic performances and reduce the occurrence of indirect injuries.

WO 2018/036495 A1 discloses a method for assessing exercise performance comprising endurance, power, risk of soft tissue injury and characterized by detecting of polymorphic sites from a subject's sample including COL5A1 rs12722, HFE rs1799945, ACTN3 rs1815739 and MCT1 rs1049434 and determining an individualized assessment by statistical analysis.

WO 2011/094815 A1 discloses a method for determining a genetic predisposition of a subject to an exercise performance trait, by assaying a genetic sample from the subject for a plurality of polymorphisms, such as ACE, ACTN3, ADBR2, AMPD1, CKM, EPAS1, GDF-8, HFE, NFATC4, NOS3, NRF2, PPARa, PPARS, PPARyCIa, PPARyCl , TFAM, UCP2, UCP3 and VEGFA.

### Summary of the Invention

It is an object of the present invention to provide a method to predict the predisposition in a human individual to muscular and/or ligament injuries.

This object is achieved by means of the method as defined in claim 1.

### Brief Description of the Drawings

Figure 1 shows the distribution and frequency of Performance Enhancing Polymorphisms in SSC Naples Football players. (A-E) Genes, alleles and genotype frequency in the 32 SSC Naples football players. In black the homozygous wild type genotype, in light grey the heterozygous and in dark grey the homozygous polymorphic genotype.
Figure 2 shows the analysis of athletic performances and injury rate of SSC Naples athletes (adapted from Ranking Serie A-Report Fisico (A) and UEFA elite club injury study (B, C)).

### Detailed Description of the Invention

The method for predicting the predisposition to muscular and/or ligament injuries in a human individual according to the present invention comprises the following steps.

First, a genetic sample obtained from the human individual is assayed for the presence of at least one genetic variation in each of the following genes:
ACTN3 Gene ID: 89; SNP Accession number: rs1815739
COL5A1 Gene ID: 1289; SNP Accession number: rs12722
VEGF Gene ID: 7422; SNP Accession number: rs2010963
MCT1 Gene ID: 6566; SNP Accession number: rs1049434
HFE Gene ID: 3077; SNP Accession number: rs1799945 to obtain a polymorphism profile.

Then, a regression function associated to the polymorphism profile is determined.

Finally, the individual's predisposition to muscular and/or ligament injuries is classified on the basis of the value of said regression function.

The human individual is preferably an athlete, more preferably a soccer player.

The 5 genes ACTN3, COL5A1, VEGF, MCT1, and HFE were identified by mining literature for genes previously correlated to athletic performances, such as endurance capacity, lactic acid clearance, iron intake and clearance, muscle fatigue.

ACTN3 encodes the a-actinin 3, a protein responsible for fast muscle fibers functioning (Lee et al., 2016). The ACTN3 R577X polymorphism is suspected, since several years (Yang et al., 2003), to be associated to particular athletic performances in power-oriented elite athletes of different ethnicities. This polymorphism is, at genomic level, a C to T transition in position 18705 and, at protein level an Arginine to X mutation in position 577 (R577X) . The polymorphism can be present in homozygosis (DNA: TT or protein: XX), in heterozygosis (DNA: CT or Protein: RX) or absent (DNA: CC or protein: RR). The CC (RR at protein level) and CT (RX at protein level) genotypes were initially found increased in frequency in elite strength athletes, in agreement with the fact that the TT (XX) mutation was previously associated with impaired muscle performance. (Roth et al., 2008). The enrichment in CC and CT genotypes, however, was not confirmed by different large-scale studies (Massidda et al., 2015b; Rankinen et al., 2016) casting doubts on the original interpretation and on the differences between elite athletes and the general population. However, the exact genotype indeed plays a role in differentiating among athletes of the same discipline, since in a study on 200 Brazilian football players, the presence of a CC genotype was associated with players fastest in short distances and with higher jump potential, while the TT genotype was associated with higher aerobic capacity (Pimenta et al., 2013). Very recently, the TT genotype has been robustly associated with a higher incidence of muscle injuries (Massidda et al., 2017).

The genetic variation of ACTN3 is a C to T polymorphism in position 18705 of the gene.

COL5A1 encodes the α1 chain of type V collagen, mainly found in ligaments and tendons and associated with severity of musculoskeletal injuries (Massidda et al., 2015a). A C to T single nucleotide polymorphism (rs12722) in the 3' untranslated region of COL5A1 gene improves mRNA stability and, in turn, protein production resulting in an increased amount of type V collagen (Abrahams et al., 2014; Bertuzzi et al., 2014). This increase in Collagen V is capable of altering the mechanical properties of tendons and may result in an increased predisposition to tendons and ligaments injuries.

The genetic variation of COL5A1 is a C to T polymorphism in position 205765 of the gene.

MCT1 encodes a lactate transporter (Merezhinskaya et al., 2000) and is responsible of the uptake of lactate by Type I oxidative fibers. Its activity has the double beneficial effect of: i) reducing the circulating levels of lactate and ii) increasing the levels of lactate in the muscle fibers that can use it as fuel. Interestingly MCT1 expression levels are increased upon high-intensity trainings, enforcing its role in enhancing the athletic performances through lactate reabsorption. A performance enhancing polymorphism (rs1049434) in the MCT1 gene has been identified in position 47005 of the gene, corresponding to an A to T transversion which results in a Glutamic acid to Aspartic acid substitution in position 490 of the protein (Glu490Asp). Compared to carriers of the A allele, the presence of the minor allele (T) is causative of a 2-fold reduction in lactate transport by MCT1 (Merezhinskaya et al., 2000) and has been correlated with higher levels of blood lactate during high-intensity training (Cupeiro et al., 2010). These findings highlight the impact that MCT1 A>T polymorphism may have on endurance performances and on athlete's response to specific training. It has been demonstrated however that the frequency of the T allele of MCT1 was high in elite sprinters, suggesting that reduced lactate uptake by type I oxidative fibers is not adverse to anaerobic activities (Kikuchi et al., 2017; Sawczuk et al., 2015). By contrast, the T-allele may be deleterious for the aerobic and endurance activities (such as football playing).

The genetic variation of MCT1 is an A to T polymorphism in position 47005 of the gene.

VEGF encodes the Vascular Endothelial Growth Factor, associated with an improvement of aerobic performances (Prior et al., 2006). Variations of the DNA sequence in the promoter of VEGF have been correlated with different levels of VEGF expression. It has been reported that a C to G polymorphism in the VEGF promoter at position 5398 of the gene is causative, when in homozygosis (GG), of a reduced expression of VEGF in hypoxic conditions and this causes, in turn, a reduced maximal oxygen consumption. However, it has also been demonstrated that whenever the polymorphism occurs in heterozygosis (CG) the VEGF expression under hypoxia are null and VO2max (training-induced increased oxygen consumption) is not affected.

The genetic variation of VEGF is a C to G polymorphism in position 5398 of the gene.

HFE (High Fe; hereditary haemochromatosis gene) encodes a transmembrane protein involved in blood and tissue iron homeostasis (Luszczyk et al., 2017). Although HFE is not an iron transporter, it interacts and regulates the activity of proteins, such as the transferrin receptor (being a negative regulator of it), that directly controls iron uptake from blood circulation, and the ferroportin, a transmembrane protein that clears iron from the cell excreting it into the blood. HFE is thus able to control the homeostasis of iron (Barton et al., 2015). Loss of function of HFE results in a genetic disease known as haemochromatosis, characterized by excessive accumulation of iron into the organism. However, high iron content, below the pathological threshold, may be beneficial for athletic performances. The HFE C187G polymorphism results, at protein level, in the substitution of a Histidine with an Aspartic acid in position 63 (H63D; rs1799945) with the subsequent reduction of HFE function. HFE H63D has a reduced ability to bind to transferrin receptor, thus resulting in an increased transport of iron into blood and cells. Although a clear association of HFE H63D polymorphism with elite athletic performances has not been established, it is worth to note that in several studies a higher frequency of HFE H63D has been found in endurance athletes such as cyclists (Chicharro et al., 2004; Deugnier et al., 2002). Iron is extremely important for oxygen transport and delivery to the muscles; thus it is reasonable that any impairment of iron uptake would result in an increase of circulating iron and an increase in oxygen availability, conditions that are both important for the execution of aerobic performances.

The genetic variation of HFE is a C to G polymorphism in position 8671 of the gene.

According to the invention, the combination of the C>T polymorphism in position 18705 of ACTN3 gene, of the C>T polymorphism in position 205765 of COL5A1 gene; of the A>T polymorphism in position 47005 of MCT1 gene; of the C>G polymorphism in position 5398 of VEGF gene, of the C>G polymorphism in position 8671 of HFE gene is associated, through a regression function, with muscular and/or ligament injuries.

In the method according to the present invention, the regression function is preferably a logistic regression function of the form: Log(P(Response=n injuries)/P(Response=not n injuries)) =- intercept+genotype1*A+genotype2*B+... . .genotypeN+X

In particular, the appropriate coefficients (capital letters in the previous equation) and the appropriate genotypes are reported vs. the number of muscular injuries in the following table:

| | Source | Value | |
|---|---|---|---|
| Injuries | | | Standard error |
| 1 | Intercept | 1,623 | 106,189 |
| | HFE H63D C>G-CC | 0 | 0 |
| | HFE H63D C>G-CG | -48,358 | 439,427 |
| | COL5A1 C>T-CC | 0 | 0 |
| | COL5A1 C>T-CT | 5,539 | 106,915 |
| | COL5A1 C>T-TT | -8,301 | 108,771 |
| | ACTN3 C>T-CC | 0 | 0 |
| | ACTN3 C>T-CT | -31,124 | 118,958 |
| | ACTN3 C>T-TT | 15,274 | 113,562 |
| | VEGF C>G-CC | 0 | 0 |
| | VEGF C>G-CG | -29,084 | 121,93 |
| | VEGF C>G-GG | 5,595 | 107,544 |
| | MCT1 T>A-AA | 0 | 0 |
| | MCT1 T>A-TA | 20,488 | 46,845 |
| | MCT1 T>A-TT | 8,544 | 238,037 |
| 2 | Intercept | -8,952 | 157,227 |
| | HFE H63D C>G-CC | 0 | 0 |
| | HFE H63D C>G-CG | -18,898 | 635,308 |
| | COL5A1 C>T-CC | 0 | 0 |
| | COL5A1 C>T-CT | 5,473 | 214,995 |
| | COL5A1 C>T-TT | 0,146 | 135,185 |
| | ACTN3 C>T-CC | 0 | 0 |
| | ACTN3 C>T-CT | -38,322 | 236,481 |
| | ACTN3 C>T-TT | -19,458 | 528,236 |
| | VEGF C>G-CC | 0 | 0 |
| | VEGF C>G-CG | -35,13 | 522,638 |
| | VEGF C>G-GG | 1,664 | 510,9 |
| | MCT1 T>A-AA | 0 | 0 |
| | MCT1 T>A-TA | 34,86 | 531,118 |
| | MCT1 T>A-TT | 30,601 | 648,352 |
| 3 | Intercept | -9,873 | 265,097 |
| | HFE H63D C>G-CC | 0 | 0 |
| | HFE H63D C>G-CG | 10,767 | 229,872 |
| | COL5A1 C>T-CC | 0 | 0 |
| | COL5A1 C>T-CT | 7,262 | 252,038 |
| | COL5A1 C>T-TT | -4,266 | 272,507 |
| | ACTN3 C>T-CC | 0 | 0 |
| | ACTN3 C>T-CT | -5,608 | 196,801 |
| | ACTN3 C>T-TT | -14,486 | 354,511 |
| | VEGF C>G-CC | 0 | 0 |
| | VEGF C>G-CG | -4,236 | 322,011 |
| | VEGF C>G-GG | 8,283 | 275,336 |
| | MCT1 T>A-AA | 0 | 0 |
| | MCT1 T>A-TA | 2,783 | 152,812 |
| | MCT1 T>A-TT | -10,311 | 202,873 |
| 4 | Intercept | -25,551 | 392,012 |
| | HFE H63D C>G-CC | 0 | 0 |
| | HFE H63D C>G-CG | -26,405 | 6744,682 |
| | COL5A1 C>T-CC | 0 | 0 |
| | COL5A1 C>T-CT | 21,586 | 369,35 |
| | COL5A1 C>T-TT | 0,754 | 305,22 |
| | ACTN3 C>T-CC | 0 | 0 |
| | ACTN3 C>T-CT | -2,625 | 201,156 |
| | ACTN3 C>T-TT | -1,426 | 215,06 |
| | VEGF C>G-CC | 0 | 0 |
| | VEGF C>G-CG | -4,072 | 208,386 |
| | VEGF C>G-GG | -0,416 | 337,437 |
| | MCT1 T>A-AA | 0 | 0 |
| | MCT1 T>A-TA | 18,238 | 315,258 |
| | MCT1 T>A-TT | -2,07 | 268,887 |

The method preferably comprises assaying the genetic sample obtained from the human individual for the presence of at least one genetic variation in at least one other gene to obtain a polymorphism profile. Examples of such genes are ACEI, AMPD1, CKMM1, GDF8, PPARGC1A, mtND5, IGF1, IGFBP3, DRD2/ANKK1, SVIL, SLC22A3, NRP2, TTN, H19, ID3, MIPEP, CPVL, DEPDC6, BTAF1, DIS3L.

### Examples

The present inventors carried out a study with the medical staff of the SSC Napoli, in which 32 elite football players, subjected to the standard sport medical evaluation and practices, were also screened for genetic polymorphism in 5 key genes (ACTN3, COL5A1, MCT1, VEGF, HFE) that, at different extent, have been correlated to athletic performances and to injuries predisposition.

The screening for the genetic polymorphisms in the 5 genes was carried out by standard techniques such as PCR amplification of genomic DNA followed by sequencing or real-time PCR.

The analysis of the polymorphisms in the above said 5 genes represents the central point of a multidisciplinary method that can be adopted by elite football teams to obtain an improvement of athletic performances and a concomitant reduction of injuries by tailoring training and nutritional programs to the genetic background of each athlete. The genetic fingerprinting of single athletes led the inventors to the identification of two performance enhancing polymorphisms (ACTN3 18705C>T and VEGF-634C>G) significantly enriched in the SSC Naples football players. Moreover, a genetic model was devised based on the polymorphisms in the entire gene set analyzed, which was used to reduce the athlete predisposition to injuries by dictating a personalized nutrition and training program.

The usefulness of such approach is concordant with data showing that the SSC Napoli football team has been recently found as the healthiest and least injured team in Europe, while covering the highest distance/match with the highest number of high intensity actions/match.

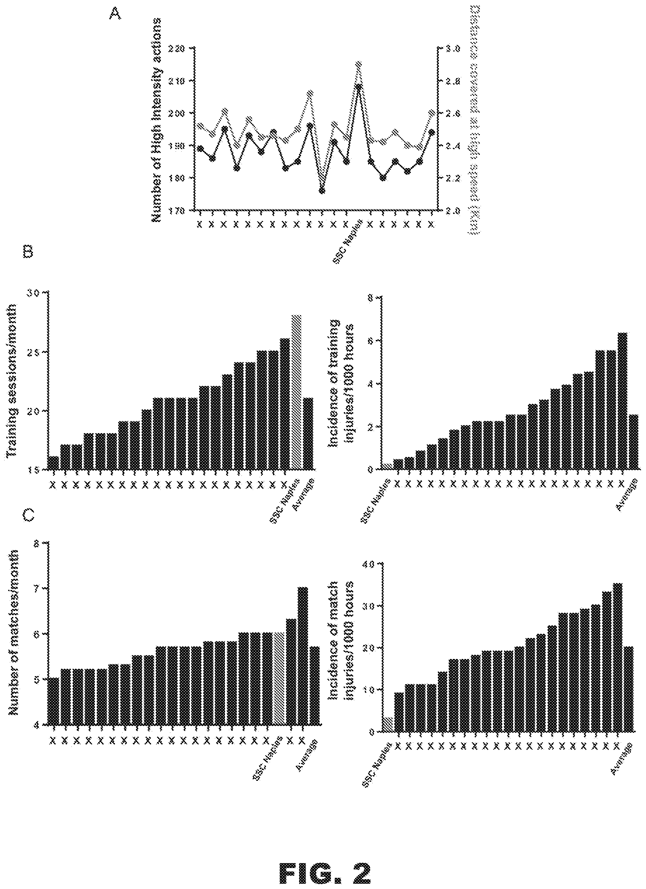

More in particular, Figure 2A shows a graph reporting the athletic performances in the 38 matches of the Italian championship 2017-2018. The black line indicates the number of high intensity actions and the grey line indicates the distance covered at high speed (>15km/h) by the 20 teams of Serie A, B, C. The graph presented are adapted from the mid-season report (2017-2018) of the UEFA Elite club injury study. Figure 2B shows data regarding the number of training sessions/month and the incidence of injuries/1000hours of training. It is clear that SSC Naples is the Team, among the other participants to the UEFA Champions League, with the highest number of training session/month and with the lowest incidence of indirect injuries. Figure 2C shows data regarding the number of matches/month and the incidence of injuries/1000hours of match. It is clear that SSC Naples is one of the teams with the highest number of matches/month and with the lowest incidence of indirect injuries.

### Example 1: ACTN3

The SSC Naples football team presents a high frequency of TT homozygotes, which is statistically different from controls and athletes in all the above-mentioned studies. In line with results reported for football players (Pimenta et al., 2013) this might indicate that, on average, SSC Naples athletes have higher aerobic capacity. Furthermore, very recently, the TT genotype has been robustly associated with a higher incidence of muscle injuries (Massidda et al., 2017). Considering these last findings, the SSC Naples medical staff designed personalized training programs for the players with the ACTN3 TT genotype, aiming to reduce the risk of muscular injuries for these players. The overall athletic performances of SSC Naples, recognized by the Lega Calcio Serie A, agreed with a possible effect of the optimized training program. (Fig. 2A).

### Example 2:

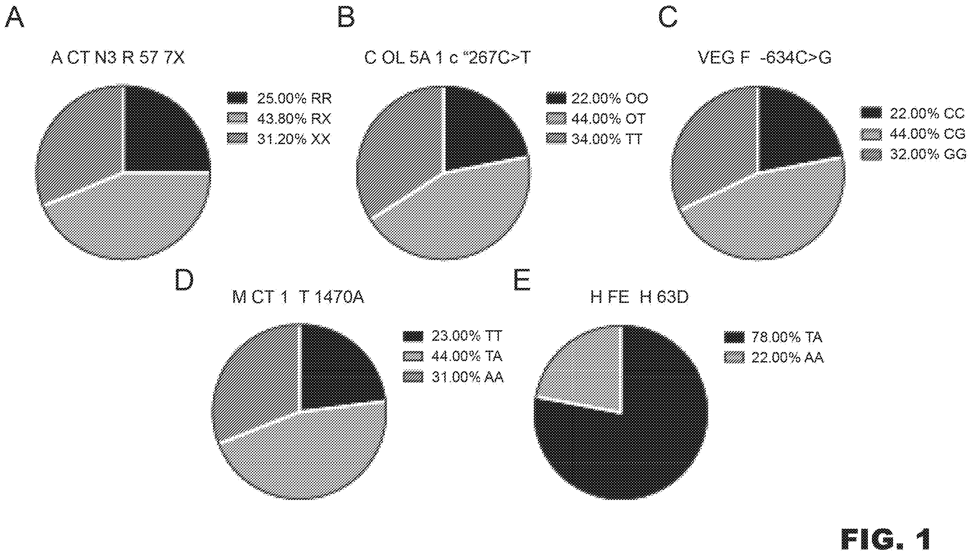

COL5A1 rs12722 polymorphism (C to T) distribution in the cohort analyzed in this study highlights a prevalence of heterozygous (CT) genotype, that accounts for the majority of athletes (44%) compared to the homozygous genotypes CC (22%) and TT (34%) (Fig.1B). In the case of this polymorphism, small excesses in the frequency of the CC allele have been associates with better performances in a large controlled study of rugby players (Heffernan et al., 2017). The frequency of CC genotype in the Naples football players is similar to that of the professional rugby players included in the study by Heffernan and colleagues. However, the number of athletes included in the present study is too limited for the detection of the expected small effect (insufficient sample power), so that is not possible to draw any significant conclusion on whether the Naples football players deviates significantly from the general population (i.e. the control sample reported in the study by Heffernan et al., 2017).

### Example 3: VEGF

The SSC Naples 32 football players were screened for the rs2010963 polymorphism (-634C>G). The three possible genotypes CC, CG, GG are represented as follows. The heterozygous (CG) is the single most diffuse genotype (46%), as compared to the homozygous genotypes CC (22%) and GG (32%) (Fig.1C). In a large study on 670 Russian athletes of different disciplines, a small but significant excess of the C allele has been correlated to higher aerobic performance of athletes and to a better lactic acid metabolism (Akhmetov et al., 2008). As for the case of the ACTN gene, SSC Naples football players exhibit a significant excess of haplotype conferring better aerobic capacities, with the CC homozygotes exceeding in a statistically significant proportion the frequency measured both for the control and the athlete populations in the Russian study, indirectly confirming the association the of G-634C polymorphism with physical performances of the athletes. This genetic makeup of the team agrees with the fact that the SSC Naples has been the Italian football team which covered the highest distance/match with the highest number of high intensity actions/match (Fig. 2A). These evidences support the tight connection that is needed among athletes, medical staff and geneticist in order to allow a tailored training program for each athlete that takes into account both the physical and genetic features.

### Example 4: MCT1

The occurrence of MCT1 A1470T polymorphism was analysed in the 32 SSC Naples football players; the three possible genotypes AA, AT, TT turned to be distributed with a prevalence of heterozygous (AT) genotype, that accounts for most athletes (46%) as compared to the homozygous genotypes AA (23%) and GG (31%) (Fig. 1D). In a large study on 323 Russian athletes and 467 control individuals, a small excess of the AA genotype frequency in the athlete population has been associated with higher in endurance (Fedotovskaya et al., 2014). However, the limited sample used in the present study did not allow the inventors to find any significant difference between the AA frequency among the Naples football players and the control population used in that study, thus preventing any conclusion to be drawn. However, in a recent study on a population on 73 male football players, the AA genotype has been found associated to a higher risk of muscular injuries (Massidda et al., 2015c). The frequency of the AA genotype found in the published study involving 73 football players is statistically identical to that observed in the Naples team, meaning that the genetic risk of muscular problems due to this variant is not different in the Naples football team as compared to other teams in the same country. Thus, the observed lower frequency of muscular accidents for this team must be due to reasons other than the genetic MCT1 makeup. Among the possible reasons, the superior performances of the studied team can be connected to the special training programs and nutritional intervention (i.e. specific formulation of sport drinks to carbohydrate and fluids supplementation) developed by the medical staff of SSC Naples with the help of professional nutritionists to improve the athletic performances of the athletes (Fig.2A).

### Example 5: HFE

The HFE H63D polymorphism, exclusively in heterozygosis with the wild type allele (CG genotype) has been found in 22% of the SSC Naples football players (Fig. 1E). The players carrying the HFE H63D polymorphism have been carefully followed by the medical staff of SSC Naples that periodically checked the serum iron, transferrin and ferritin levels in order to control the iron homeostasis and execute nutritional interventions to avoid the variation of these parameters above a certain threshold detrimental for the health and the performance of the athletes. For the HFE polymorphism, a difference was obtained which is not significant with the data reported for a large, general population study and for a smaller control population in a different study (Chicharro et al., 2004; Katsarou et al., 2016). By contrast, the G haplotype resulted significantly less present in Naples athletes than in a sample of 65 endurance athletes, with a significant difference between the overall number of individuals carrying at least one H63D C>G mutation (P = 0.0067; Chi-squared test as recommended by (Campbell, 2007) and (Richardson, 2011). In this respect, it was concluded that Naples football players do not differ from the general control population.

### Example 6: Combined analysis

From the single-gene analysis discussed so far, it turned out that the SSC Naples team is predicted to be negatively influenced at the genetic level by the measured frequency of ACTN3 haplotypes, which predisposes the players to muscular injuries, and by specific MCT1 haplotypes positively affecting the iron transport, which turned out to be less abundant in comparison to other teams playing in Italy. However, the team is also well disposed by its VEGF superior genetic makeup, predicted to confer high aerobic capacities and in agreement with the reported team's highest distance/match covered and highest number of high intensity actions/match performed.

A different question to be answered was whether the combined effect of the 5 genes included in the analysis could explain the individual predisposition of the single football players to muscular and/or ligament injuries. The target variable considered for the analysis (i.e. the quantity to be predicted) was the overall amount of injuries occurring to a given player during the season 2014-2018, and as predictor its genetic makeup at the 5 considered genes.

The overall number of muscular injuries occurred to each player is reported in the following table.

| **Player** | **Injuries occurred** | **Player** | **Injuries occurred** |
|---|---|---|---|
| 1.xxxxx | 0 | 16.xxxxx | 2 |
| 2.xxxxx | 0 | 17.xxxxx | 0 |
| 3.xxxxx | 1 | 18.xxxxx | 0 |
| 4.xxxxx | 3 | 19.xxxxx | 1 |
| 5.xxxxx | 0 | 20.xxxxx | 0 |
| 6.xxxxx | 0 | 21.xxxxx | 4 |
| 7.xxxxx | 2 | 22.xxxxx | 1 |
| 8.xxxxx | 0 | 23.xxxxx | 0 |
| 9.xxxxx | 1 | 24.xxxxx | 0 |
| 10.xxxxx | 0 | 25.xxxxx | 0 |
| 11.xxxxx | 0 | 26.xxxxx | 0 |
| 12.xxxxx | 1 | 27.xxxxx | 0 |
| 13.xxxxx | 1 | 28.xxxxx | 0 |
| 14.xxxxx | 0 | 29.xxxxx | 2 |
| 15.xxxxx | 1 | 30.xxxxx | 0 |

Complete genetic information was available only for 27 players. Assuming as regression variable the number of muscular incidents, a multinomial fitting of a logistic model based on the players genotype was performed. After the regression, an almost complete separation of observations was detected, with the following very good determination coefficients: R²(McFadden)= 0.953, R²(Cox and Snell) = 0.876, R²(Nagelkerke) = 0,986. The goodness of the fit is demonstrated by a Chi-square = 56.446; with the available degrees of freedom, the probability of finding a value of Chi-square equal or superior in case the null hypothesis is true (i.e. there is no correlation between the genetic makeup and the number of injuries for any given player) is p=0,016, thus below the usual significance alpha threshold of p=0.05. In agreement with such a good and significant fitting, the players' reclassification table obtained by applying the identified model to the 27 considered players is the following:

| from \ to | 0 | 1 | 2 | 3 | 4 | Total | % correct |
|---|---|---|---|---|---|---|---|
| 0 | **16** | 0 | 0 | 0 | 0 | 16 | 100,00% |
| 1 | 1 | **6** | 0 | 0 | 0 | 7 | 85,71% |
| 2 | 0 | 0 | **2** | 0 | 0 | 2 | 100,00% |
| 3 | 0 | 0 | 0 | **1** | 0 | 1 | 100,00% |
| 4 | 0 | 0 | 0 | 0 | **1** | 1 | 100,00% |
| Total | 17 | 6 | 2 | 1 | 1 | 27 | 97,14% |

As evident from the table, the prediction of the number of injuries based on the obtained model is wrong in only a single case (a player which was injured only once is back-predicted to have incurred in no injuries).

This is the most accurate athletic injuries predictive model known so far, relying on the thoughtful selections of 5 genes already involved in athletic performances, which were however never tested simultaneously for their predictive power. With the help of this model, the SSC Naples medical staff or the staff of other football teams can now in principle focus their efforts on players most at risk, tuning their training programs and giving them useful insights, so to optimize the performances of the athletes.

### Advantages

As can be seen from the Examples, among the 5 genes selected for this study, ACTN3, COL5A1 and VEGF influence the athletic performances and the indirect injury predisposition, independently of any nutritional supply. The effect of these performance enhancing polymorphisms may be enhanced or counteracted only by developing a personalized training. For other two genes, MCT1 and HFE, in which performance enhancing polymorphisms have been identified, a nutritional intervention along with the development of specific training programs may be undertaken to counteract their negative or enhance their positive influences.

Moreover, it has been shown that the combination of the five genes has a strong predictive power. The obtained regression indicates that the identified 5 genes-performance association could, at least in part, explain why an athlete is more susceptible to injuries than another one, so that, it can be used to identify athletes which need special cares during training sessions and special attention when taking the field and could support the development of tailored injury prevention programs and/or targeted therapeutic interventions.

## Claims

1. A method to predict the predisposition to muscular and/or ligament injuries in a human individual comprising:
a) assaying a genetic sample obtained from the human individual for the presence of at least one genetic variation in each of the following genes: ACTN3, COL5A1, MCT1, VEGF, HFE, to obtain a polymorphism profile;
b) determining a regression function associated to the polymorphism profile;
c) classifying the individual's predisposition to muscular and/or ligament injuries on the basis of the value of said regression function, wherein:
- the genetic variation of ACTN3 is a C to T polymorphism in position 18705 of the gene corresponding to SNP rs1815739;
- the genetic variation of COL5A1 is a C to T polymorphism in position 205765 of the gene corresponding to SNP rs12722;
- the genetic variation of MCT1 is an A to T polymorphism in position 47005 of the gene corresponding to SNP rs1049434;
- the genetic variation of VEGF is a C to G polymorphism in position 5398 of the gene corresponding to SNP rs2010963;
- the genetic variation of HFE is a C to G polymorphism in position 8671 of the gene corresponding to SNP rs1799945.

2. The method according to claim 1, wherein the regression function is a logistic regression function of the form: Log(P (Response=n injuries)/P(Response=not n injuries)) =- intercept+genotype1*A+genotype2*B+... . .genotypeN+X wherein the appropriate coefficients (capital letters in the previous equation) and the appropriate genotypes are reported vs. the number of muscular injuries in the following table:
| | Source | Value | |
|---|---|---|---|
| Injuries | | | Standard error |
| 1 | Intercept | 1,623 | 106,189 |
| | HFE H63D C>G-CC | 0 | 0 |
| | HFE H63D C>G-CG | -48,358 | 439,427 |
| | COL5A1 C>T-CC | 0 | 0 |
| | COL5A1 C>T-CT | 5,539 | 106,915 |
| | COL5A1 C>T-TT | -8,301 | 108,771 |
| | ACTN3 C>T-CC | 0 | 0 |
| | ACTN3 C>T-CT | -31,124 | 118,958 |
| | ACTN3 C>T-TT | 15,274 | 113,562 |
| | VEGF C>G-CC | 0 | 0 |
| | VEGF C>G-CG | -29,084 | 121,93 |
| | VEGF C>G-GG | 5,595 | 107,544 |
| | MCT1 T>A-AA | 0 | 0 |
| | MCT1 T>A-TA | 20,488 | 46,845 |
| | MCT1 T>A-TT | 8,544 | 238,037 |
| 2 | Intercept | -8,952 | 157,227 |
| | HFE H63D C>G-CC | 0 | 0 |
| | HFE H63D C>G-CG | -18,898 | 635,308 |
| | COL5A1 C>T-CC | 0 | 0 |
| | COL5A1 C>T-CT | 5,473 | 214,995 |
| | COL5A1 C>T-TT | 0,146 | 135,185 |
| | ACTN3 C>T-CC | 0 | 0 |
| | ACTN3 C>T-CT | -38,322 | 236,481 |
| | ACTN3 C>T-TT | -19,458 | 528,236 |
| | VEGF C>G-CC | 0 | 0 |
| | VEGF C>G-CG | -35,13 | 522,638 |
| | VEGF C>G-GG | 1,664 | 510,9 |
| | MCT1 T>A-AA | 0 | 0 |
| | MCT1 T>A-TA | 34,86 | 531,118 |
| | MCT1 T>A-TT | 30,601 | 648,352 |
| 3 | Intercept | -9,873 | 265,097 |
| | HFE H63D C>G-CC | 0 | 0 |
| | HFE H63D C>G-CG | 10,767 | 229,872 |
| | COL5A1 C>T-CC | 0 | 0 |
| | COL5A1 C>T-CT | 7,262 | 252,038 |
| | COL5A1 C>T-TT | -4,266 | 272,507 |
| | ACTN3 C>T-CC | 0 | 0 |
| | ACTN3 C>T-CT | -5,608 | 196,801 |
| | ACTN3 C>T-TT | -14,486 | 354,511 |
| | VEGF C>G-CC | 0 | 0 |
| | VEGF C>G-CG | -4,236 | 322,011 |
| | VEGF C>G-GG | 8,283 | 275,336 |
| | MCT1 T>A-AA | 0 | 0 |
| | MCT1 T>A-TA | 2,783 | 152,812 |
| | MCT1 T>A-TT | -10,311 | 202,873 |
| 4 | Intercept | -25,551 | 392,012 |
| | HFE H63D C>G-CC | 0 | 0 |
| | HFE H63D C>G-CG | -26,405 | 6744,682 |
| | COL5A1 C>T-CC | 0 | 0 |
| | COL5A1 C>T-CT | 21,586 | 369,35 |
| | COL5A1 C>T-TT | 0,754 | 305,22 |
| | ACTN3 C>T-CC | 0 | 0 |
| | ACTN3 C>T-CT | -2,625 | 201,156 |
| | ACTN3 C>T-TT | -1,426 | 215,06 |
| | VEGF C>G-CC | 0 | 0 |
| | VEGF C>G-CG | -4,072 | 208,386 |
| | VEGF C>G-GG | -0,416 | 337,437 |
| | MCT1 T>A-AA | 0 | 0 |
| | MCT1 T>A-TA | 18,238 | 315,258 |
| | MCT1 T>A-TT | -2,07 | 268,887 |

3. The method according to claim 1 or 2, wherein the method comprises assaying the genetic sample obtained from the human individual for the presence of at least one genetic variation in at least one other gene to obtain a polymorphism profile.

## Patentansprüche

1. Verfahren, um die Anfälligkeit für Muskel- und/oder Bänderverletzungen bei einem menschlichen Individuum vorherzusagen, umfassend:
a) Untersuchen einer genetischen Probe, die von dem menschlichen Individuum erhalten wird, auf das Vorhandensein von mindestens einer genetischen Variation in jedem der folgenden Gene: ACTN3, COL5A1, MCT1, VEGF, HFE, um ein Polymorphismusprofil zu erhalten;
b) Bestimmen einer Regressionsfunktion, die dem Polymorphismusprofil zugeordnet ist;
c) Klassifizieren der Anfälligkeit des Individuums für Muskel- und/oder Bänderverletzungen auf der Basis des Wertes der Regressionsfunktion, wobei:
- die genetische Variation von ACTN3 ein C-zu-T-Polymorphismus an Position 18705 des Gens ist, das SNP rs1815739 entspricht;
- die genetische Variation von COL5A1 ein C-zu-T-Polymorphismus an Position 205765 des Gens ist, das SNP rs12722 entspricht;
- die genetische Variation von MCT1 ein A-zu-T-Polymorphismus an Position 47005 des Gens ist, das SNP rs1049434 entspricht;
- die genetische Variation von VEGF ein C-zu-G-Polymorphismus an Position 5398 des Gens ist, das SNP rs2010963 entspricht;
- die genetische Variation von HFE ein C-zu-G-Polymorphismus an Position 8671 des Gens ist, das SNP rs1799945 entspricht.

2. Verfahren nach Anspruch 1, wobei die Regressionsfunktion eine logistische Regressionsfunktion der Form ist: Log(P(Reaktion=n Verletzungen)/P(Response=keine n Verletzungen)) =- Schnittpunkt+Genotyp1*A+Genotyp2*B+... ••GenotypN+X wobei die dazugehörigen Koeffizienten (Großbuchstaben in der vorstehenden Gleichung) und die dazugehörigen Genotypen gegenüber der Anzahl von Muskelverletzungen in der folgenden Tabelle aufgeführt sind:
| | Quelle | Wert | |
|---|---|---|---|
| Verletzungen | | | Standardfehler |
| 1 | Schnittpunkt | 1,623 | 106,189 |
| | HFE H63D C>G-CC | 0 | 0 |
| | HFE H63D C>G-CG | -48,358 | 439,427 |
| | COL5A1 C>T-CC | 0 | 0 |
| | COL5A1 C>T-CT | 5,539 | 106,915 |
| | COL5A1 C>T-TT | -8,301 | 108,771 |
| | ACTN3 C>T-CC | 0 | 0 |
| | ACTN3 C>T-CT | -31,124 | 118,958 |
| | ACTN3 C>T-TT | 15,274 | 113,562 |
| | VEGF C>G-CC | 0 | 0 |
| | VEGF C>G-CG | -29,084 | 121,93 |
| | VEGF C>G-GG | 5,595 | 107,544 |
| | MCT1 T>A-AA | 0 | 0 |
| | MCT1 T>A-TA | 20,488 | 46,845 |
| | MCT1 T>A-TT | 8,544 | 238,037 |
| 2 | Schnittpunkt | -8,952 | 157,227 |
| | HFE H63D C>G-CC | 0 | 0 |
| | HFE H63D C>G-CG | -18,898 | 635,308 |
| | COL5A1 C>T-CC | 0 | 0 |
| | COL5A1 C>T-CT | 5,473 | 214,995 |
| | COL5A1 C>T-TT | 0,146 | 135,185 |
| | ACTN3 C>T-CC | 0 | 0 |
| | ACTN3 C>T-CT | -38,322 | 236,481 |
| | ACTN3 C>T-TT | -19,458 | 528,236 |
| | VEGF C>G-CC | 0 | 0 |
| | VEGF C>G-CG | -35,13 | 522,638 |
| | VEGF C>G-GG | 1,664 | 510,9 |
| | MCT1 T>A-AA | 0 | 0 |
| | MCT1 T>A-TA | 34,86 | 531,118 |
| | MCT1 T>A-TT | 30,601 | 648,352 |
| 3 | Schnittpunkt | -9,873 | 265,097 |
| | HFE H63D C>G-CC | 0 | 0 |
| | HFE H63D C>G-CG | 10,767 | 229,872 |
| | COL5A1 C>T-CC | 0 | 0 |
| | COL5A1 C>T-CT | 7,262 | 252,038 |
| | COL5A1 C>T-TT | -4,266 | 272,507 |
| | ACTN3 C>T-CC | 0 | 0 |
| | ACTN3 C>T-CT | -5,608 | 196,801 |
| | ACTN3 C>T-TT | -14,486 | 354,511 |
| | VEGF C>G-CC | 0 | 0 |
| | VEGF C>G-CG | -4,236 | 322,011 |
| | VEGF C>G-GG | 8,283 | 275,336 |
| | MCT1 T>A-AA | 0 | 0 |
| | MCT1 T>A-TA | 2,783 | 152,812 |
| | MCT1 T>A-TT | -10,311 | 202,873 |
| 4 | Schnittpunkt | -25,551 | 392,012 |
| | HFE H63D C>G-CC | 0 | 0 |
| | HFE H63D C>G-CG | -26,405 | 6744,682 |
| | COL5A1 C>T-CC | 0 | 0 |
| | COL5A1 C>T-CT | 21,586 | 369,35 |
| | COL5A1 C>T-TT | 0,754 | 305,22 |
| | ACTN3 C>T-CC | 0 | 0 |
| | ACTN3 C>T-CT | -2,625 | 201,156 |
| | ACTN3 C>T-TT | -1,426 | 215,06 |
| | VEGF C>G-CC | 0 | 0 |
| | VEGF C>G-CG | -4,072 | 208,386 |
| | VEGF C>G-GG | -0,416 | 337,437 |
| | MCT1 T>A-AA | 0 | 0 |
| | MCT1 T>A-TA | 18,238 | 315,258 |
| | MCT1 T>A-TT | -2,07 | 268,887 |

3. Verfahren nach Anspruch 1 oder 2, wobei das Verfahren das Untersuchen der genetischen Probe, die von dem menschlichen Individuum erhalten wird, auf das Vorhandensein von mindestens einer genetischen Variation in mindestens einem anderen Gen umfasst, um ein Polymorphismusprofil zu erhalten.

## Revendications

1. Procédé de prédiction de la prédisposition aux blessures musculaires et/ou ligamentaires chez un individu humain comprenant :
a) l'analyse d'un échantillon génétique obtenu à partir de l'individu humain pour détecter la présence d'au moins une variation génétique dans chacun des gènes suivants : ACTN3, COL5A1, MCT1, VEGF, HFE, pour obtenir un profil de polymorphisme ;
b) la détermination d'une fonction de régression associée au profil de polymorphisme ;
c) la classification de la prédisposition de l'individu aux blessure musculaires et/ou ligamentaires sur la base de la valeur de ladite fonction de régression, dans lequel :
- la variation génétique de ACTN3 est un polymorphisme C à T en position 18705 du gène correspondant au SNP rs1815739 ;
- la variation génétique de COL5A1 est un polymorphisme C à T en position 205765 du gène correspondant au SNP rs12722 ;
- la variation génétique de MCT1 est un polymorphisme A à T en position 47005 du gène correspondant au SNP rs1049434 ;
- la variation génétique de VEGF est un polymorphisme C à G en position 5398 du gène correspondant au SNP rs2010963 ;
- la variation génétique de HFE est un polymorphisme C à G en position 8671 du gène correspondant au SNP rs1799945.

2. Procédé selon la revendication 1, dans lequel la fonction de régression est une fonction de régression logistique de la forme : Log(P(Réponse = n blessures) / P(Résponse = pas n blessures)) = - intercept + génotype1 * A + genotype2 * B +...••génotype N + X dans lequel les coefficients appropriés (lettres majuscules dans l'équation précédente) et les génotypes appropriés sont rapportés en fonction du nombre de blessures musculaires dans le tableau suivant :
| | Source | Valeur | |
|---|---|---|---|
| Blessures | | | Erreur standard |
| 1 | Intercept | 1,623 | 106,189 |
| | HFE H63D C>G-CC | 0 | 0 |
| | HFE H63D C>G-CG | -48,358 | 439,427 |
| | COL5A1 C>T-CC | 0 | 0 |
| | COL5A1 C>T-CT | 5,539 | 106,915 |
| | COL5A1 C>T-TT | -8,301 | 108,771 |
| | ACTN3 C>T-CC | 0 | 0 |
| | ACTN3 C>T-CT | -31,124 | 118,958 |
| | ACTN3 C>T-TT | 15,274 | 113,562 |
| | VEGF C>G-CC | 0 | 0 |
| | VEGF C>G-CG | -29,084 | 121,93 |
| | VEGF C>G-GG | 5,595 | 107,544 |
| | MC;T1 T>A-AA | 0 | 0 |
| | MCT1 T>A-TA | 20,488 | 46,845 |
| | MCT1 T>A-TT | 8,544 | 238,037 |
| 2 | Intercept | -8,952 | 157,227 |
| | HFE H63D C>G-CC | 0 | 0 |
| | HFE H63D C>G-CG | -18,898 | 635,308 |
| | COL5A1 C>T-CC | 0 | 0 |
| | COL5A1 C>T-CT | 5,473 | 214,995 |
| | COL5A1 C>T-TT | 0,146 | 135,185 |
| | ACTN3 C>T-CC | 0 | 0 |
| | ACTN3 C>T-CT | -38,322 | 236,481 |
| | ACTN3 C>T-TT | -19,458 | 528,236 |
| | VEGF C>G-CC | 0 | 0 |
| | VEGF C>G-CG | -35,13 | 522,638 |
| | VEGF C>G-GG | 1,664 | 510,9 |
| | MCT1 T>A-AA | 0 | 0 |
| | MCT1 T>A-TA | 34,86 | 531,118 |
| | MCT1 T>A-TT | 30,601 | 648,352 |
| 3 | Intercept | -9,873 | 265,097 |
| | HFE H63D C>G-CC | 0 | 0 |
| | HFE H63D C>G-CG | 10,767 | 229,872 |
| | COL5A1 C>T-CC | 0 | 0 |
| | COL5A1 C>T-CT | 7,262 | 252,038 |
| | COL5A1 C>T-TT | -4,266 | 272,507 |
| | ACTN3 C>T-CC | 0 | 0 |
| | ACTN3 C>T-CT | -5,608 | 196,801 |
| | ACTN3 C>T-TT | -14,486 | 354,511 |
| | VEGF C>G-CC | 0 | 0 |
| | VEGF C>G-CG | -4,236 | 322,011 |
| | VEGF C>G-GG | 8,283 | 275,336 |
| | MCT1 T>A-AA | 0 | 0 |
| | MCT1 T>A-TA | 2,783 | 152,812 |
| | MCT1 T>A-TT | -10,311 | 202,873 |
| 4 | Interception | -25,551 | 392,012 |
| | HFE H63D C>G-CC | 0 | 0 |
| | HFE H63D C>G-CG | -26,405 | 6744,682 |
| | COL5A1 C>T-CC | 0 | 0 |
| | COL5A1 C>T-CT | 21,586 | 369,35 |
| | COL5A1 C>T-TT | 0,754 | 305,22 |
| | ACTN3 C>T-CC | 0 | 0 |
| | ACTN3 C>T-CT | -2,625 | 201,156 |
| | ACTN3 C>T-TT | -1,426 | 215,06 |
| | VEGF C>G-CC | 0 | 0 |
| | VEGF C>G-CG | -4,072 | 208,386 |
| | VEGF C>G-GG | -0,416 | 337,437 |
| | MCT1 T>A-AA | 0 | 0 |
| | MCT1 T>A-TA | 18,238 | 315,258 |
| | MCT1 T>A-TT | -2,07 | 268,887 |

3. Procédé selon la revendication 1 ou 2, dans lequel le procédé comprend l'analyse de l'échantillon génétique obtenu à partir de l'individu humain pour la présence d'au moins une variation génétique dans au moins un autre gène afin d'obtenir un profil de polymorphisme.
